Europäisches Patentamt

European Patent Office

Office européen des brevets

(11) Publication number: **0 312 132 B1**

# EUROPEAN PATENT SPECIFICATION

(45) Date of publication of patent specification: **13.01.93**  (51) Int. Cl.5: **A61K 31/655**

(21) Application number: **88201797.3**

(22) Date of filing: **24.08.88**

The file contains technical information submitted after the application was filed and not included in this specification

(54) **Veterinary and human medical remedy having an antiviral and antitumorous activity, containing 2,6-diamino-3-phenyl-azo-pyridine.**

(30) Priority: **14.10.87 NL 8702446**

(43) Date of publication of application:
**19.04.89 Bulletin  89/16**

(45) Publication of the grant of the patent:
**13.01.93 Bulletin  93/02**

(84) Designated Contracting States:
**AT BE CH DE ES FR GB GR IT LI LU NL SE**

(56) References cited:

**ARCHIV FÜR HYGIENE UND BAKTERIOLOGIE, no. 145, 1961, pages 88-107; J.P. GUEPIN et al.: "Die Behandlung der Grippe und Erkältung, des Schnupfens und verschiedener anderer Viruskrankheiten bei Menschen, Tieren und Pflanzen"**

**ACTA PHARMACEUTICA FENNICA, vol. 97, no. 1, 1988, pages 29-35; A. EL-HELW et al.: "Effect of core modification on the release and bioavailability of phenazopyridine hydrochloride from ethyl cellulose-walled microcapsules"**

PHARMAZIE, vol. 39, no. 6, 1984, pages 404-406; H.M. EL-SABBAGH et al.: "Effect of some additives on the properties of phenazopyridine hydrochloride granules and their corresponding tablets"

"The Merck Index", edition 10, 1983, page 1038, no. 7083, Merck & Co., Inc., Rahway, NJ, US; "Phenazopyridine HCL"

"Martindale", edition 28, 1982, pages 272-273, no. 2685g, The Pharmaceutical Society, GB; "Phenazopyridine HCL"

(73) Proprietor: **Hak, Barend Willem
Groot Zuideveld 10
NL-4271 CC Dussen(DE)**

(72) Inventor: **van Duuren, Erris
Gevers Deynootweg 946
NL-2586 BW The Hague(NL)**

(74) Representative: **Siemens, Andreas Meinhard Ernest, Dipl.-Ing.
SIEMENS & CIE. Roskam 8
NL-4813 GZ Breda(NL)**

EP 0 312 132 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to the use of an inorganic salt of 2,6-diamino-3-phenyl-azo-pyridine (see formula I) for the preparation of a medicament for the therapy of virus-initiated sarcoma.

The base of this salt was known per se as an azo dye compound in the textile industry. Research has been carried out with regard to its antibacterial properties as a prophylactic and therapeutic compound against infections with poultry and mammals mostly in connection with the antibiotics chloroamphenicol and terramycine. At present only an additional effect of these compounds is being surmised.

Now the inorganic salts borate, carbonate, sulphate and hydrochloride of the primary compound as such are proposed for treatment of tumors. The synthesis of the active component (formula I) is known from U.S. Pat. 1,680,109. Therein the reaction of coupling of diazotized aromatic amines and $\alpha$-$\alpha$'-diaminopyridine is described, resulting in the formation of two isomers of phenyldiazo-diamino-pyridine, and separation is possible by extraction with water, wherein the mono-phenyl-isomers are more soluble than diphenylisomers. It was found that the compound is a bactericidal desinfectant.

The applicant has carried out investigations of toxic effects of the active component, including experiments with microbiological research and with chemotherapeutical research on metabolites (see formula II). The results showed, that it is acceptable, that the metabolites of the base, which are formed after administration of the component, in the body, have a considerably wider effect, specifically resulting in a striking virus-destructing activity in warm-blooded animals and even for treatment of humans, who suffer of sarcoma, which are initiated by vira.

The pharmacological and analytical results justify the admissibility that an application to humans will yield similar favourable results. After experiments in vitro this has been confirmed in vivo as well. Moreover the experiments with animals demonstrated surprisingly that there appeared also a primarily antitumoral effect against virus-initiated sarcoma, of which the haematogenic metastasis in the early stages is apprehended.

The metabolites which had been detected in urine and faeces were products of the reducing splitting of the azo-bonds, in particular the following N-heterocyclic molecules,having the general structure according to formula II, i.e. thymidine, pyrimidine and azidothymidine.

In the said formula $R_1$ and $R_2$ signify sugars, amino- and/or purine groups respectively.

Said N-heterocyclic compounds can be regarded as active components against the receptors of different virus structures.

Further metabolites of the compound in question are N-acetyl-amino-phenol, p-amino-phenol and aniline, of which a synergistic effect is surmised, but not yet evinced.

As the wall of the cells of mitochondria is an important information carrier, as described by Ashwell in "Trends of Biochemical Science", (1977), nr. 2, pages 76 a.f.and since the most dangerous virus compounds possess receptors, which can almost only be fought against by means of compounds formed in vivo, thus primarily by metabolites, it was reflected that the application of the 2,6-diamino-3-phenyl-azo-pyridine base in an appropriate form and concentration could be considered to be of major importance against virus infections of humans and of animals, while the practice of application against virus-initiated tumors and against metastases might probably be advantageous in the combat of the HIV-II-virus, the originator of Aids,and against virus Hepatitis (infectuous and serum-Hepatitis).

As the compound of the present invention has only a very moderate hepatoxic property and it has been used in small concentrations for bacterial inflammations on small scale in human medical treatment, clinical applications have been executed in cases of virus-initiated sarcoma,and these cases have recently shown considerable improvements of the symptoms of the disease, which have to be contributed to the effect of the said metabolites, or at least to one of the three first mentioned compounds of the group. Reflecting the earlier known antibacterial effect,the present compound can also be included into a broad-spectrum curative and it might be prescribed against diseases of which the diagnosis is extremely difficult, and which spread now in tropical regions.

The medical preparation for veterinary and humane treatment having antitumoral effects according to the present invention is characterized in that for oral treatment 2,6-diamino-3-phenyl-azo-pyridine-salt in a concentration of 0.5-10.0 wt./wt.% is dissolved in water.

Appropriate salts are the hydrochloride, borate and carbonate. For intramuscular application a solution of 2,6-diamino-3-phenyl-azo-pyridine-borate in a concentration in an organic solvent or in water is prepared and filled in vials of 50 ml.

For local treatment a mixture of 5.0 wt./wt.% of 2,6-diamino-3-phenyl-azo-pyridine salt in unguentum vaseline (petroleum jelly) is prepared. The administration of the preparation to mammals and to poultry is between 0.5 and 10 mg per kg body weight per day.

2

The treatment can be continued during 2 to 6 days without an interruption. The examination of the effect of the remedy showed strikingly favourable results in animals which suffered from virus diseases and which were apparently threatened by such disease.

It was also observed,that male white Wistar rats which are prone to sarcoma, could be treated prophylactically as well therapeutically with good results.

The properties of the compound for pharmacological application are the following:

Table I:

Shape: red odourless crystalline powder

melting range: 235-239°C.

Solubility:

ethanol 3.5 mg/ml

chloroform 0.4 mg/ml

diethylether 0.2 mg/ml

2-propanol 2.1 mg/ml

methanol 2.7 mg/ml

ligroin 0.1 mg/ml

water 3.2 mg/ml.

The compound can be prepared by condensation of phenazine and pyridine or by coupling of phenyl-diazonium chloride with $\alpha$-$\alpha'$-diaminopyridine in a water bath under reflux cooling, and dissolving the obtained product in boric acid, acetic acid or dilute hydro-chloric acid.

Indentification is possible, among others, by UV-spectrometry of a solution of 0.5 mg in 100 ml acidified ethanol, whereby in the range of 210-450 nm two peaks are measured at 238-240 nm ($\epsilon$ = 2.2x10$^4$) and at 390-392 nm ($\epsilon$ = 2.4x10$^4$) and three minima appear at 220 nm, 272 nm and 296 nm.

The gross formula of the hydrochloride is $C_{11}H_{11}N_5$.HCl.

The molecular weight is 249.7.

The compound does not cause haemolytic anaemia, except in overdosages, and the application against bacterial inflammations in the urethra and prostata has been cited in Martindales Extra Pharmacopoeia (Pharmaceutical Press, London),26.Ed., 1972, p.251.

Dosages up till 20 mg per kg body weight per day did not cause anaemia in cats having bacterial infections.

Toxicological data during bacterial infections have been reported in Journal of the American Veterinary Medical Association, Vol.169, (1976), no. 3, p.327-331, but the activity against tumors had not yet been recognized therewith and has not yet been published hitherto.

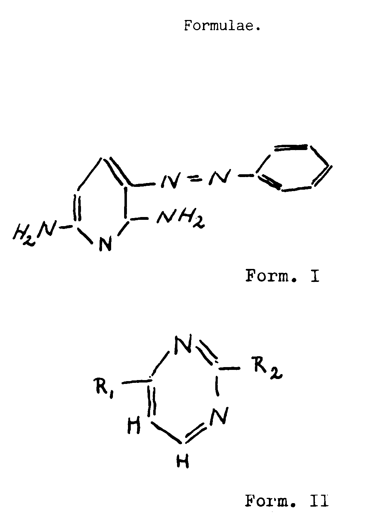

**Claims**

1. Use of 2,6-diamino-3-phenyl-azo-pyridine in the form of an inorganic salt thereof for the manufacture of a medicament for use in the treatment of sarcoma initiated by a virus.

2. Use according to claim 1, characterized in that a liquid for parenteral administration is made, comprising 1-10 % w/v of 2,6-diamino-3-phenyl-azopyridinehydrochloride, 0.1% w/v of methyl-4-hydroxybenzoate, 0.1% w/v of propyl-4-hydroxybenzoate, 5.0% w/v of polyvinylpyrrolidone, 65% w/v of N-methyl-2-pyrrolidone, and water ad inject. until 100.0% w/v.

3. Use according to claim 1, characterized in that tablets for oral administration are made, comprising 25 mg of 2,6-diamino-3-phenylazopyridine-hydrochloride, 150 mg of lactose, 70 mg maize starch, 20 mg of polyvinylpyrrolidone, 8 mg of colloidal anhydrous silica, and 8 mg of magnesium stearate.

3

4. Use according to claim 1, characterized in that ointment for local administration to the skin is made, comprising 2.0% w/v of 2,6-diamino-3-phenyl-azopyridinehydrochloride, 0.1% w/v of methyl-4-hydroxybenzoate, 0.03% w/v of propyl-4-hydroxybenzoate, 4.0% w/v of polyvinylpyrrolidone, 60.0% w/v of propyleneglycol, 1.4% w/v of hydroxyethylcellulose, and purif. water until 100.0% w/v.

**Patentansprüche**

1. Anwendung von 2,6-Diamino-3-phenyl-azo-pyridin in der Form eines anorganischen Salzes desselben zur Herstellung eines Arzneimittels zum Gebrauch bei der Behandlung von Sarkomen welche durch ein Virus initiiert wurden.

2. Anwendung nach Anspruch 1, dadurch gekennzeichnet, dass man eine Flüssigkeit zur parenteralen Verabreichung bereitet, welche 1 bis 10 Gew./Vol.% 2,6-Diamino-3-phenyl-azopyridinhydrochlorid, 0,1 Gew./Vol.% Methyl-4-hydroxybenzoat, 0,1 Gew./Vol.% Propyl-4-hydroxybenzoat, 5,0 Gew./Vol.% Polyvinyl-pyrrolidon, 65 Gew./Vol.% N-Methyl-2-pyrrolidon und Wasser ad inject. bis auf 100,0 Gew./Vol.% enthält.

3. Anwendung nach Anspruch 1, dadurch gekennzeichnet, dass man Tabletten zur oralen Verabreichung herstellt, welche 25 mg 2,6-Diamino-3-phenylazopyridin-hydrochlorid, 150 mg Laktose, 70 mg Maisstärke, 20 mg Polyvinylpyrrolidon, 8 mg kolloidale wasserfreie Kieselsäure und 8 mg Magnesiumstearat enthält.

4. Anwendung nach Anspruch 1, dadurch gekennzeichnet, dass man eine Salbe zur örtlichen Verabreichung auf der Haut herstellt, welche 2,0 Gew./Vol.% 2,6-Diamino-3-phenyl-azopyridinchlorid, 0.1 Gew./Vol.% Methyl-4-hydroxybenzoat, 0.03 Gew./Vol.% Propyl-4-hydroxybenzoat, 4.0 Gew./Vol.% Polyvinylpyrrolidon, 60.0 Gew./Vol.% Propylenglykol, 1.4 Gew./Vol.% Hydroxyäthylzellulose und gereinigtes Wasser bis auf 100.0 Gew./Vol.% enthält.

**Revendications**

1. L'emploi du 2,6-diamino-3-phényle-azopyridine en forme d'un sel inorganique de cela pour la préparation d'un médicament à l'usage pour la thérapie de sarcome initié par un virus.

2. L'emploi d'après la revendication 1, caractérisé en ce qu'un liquide pour l'administration parentérale est préparé, contenant 1-10% poids/volume du 2,6-diamino-3-phényle-azopyridine-hydrochlorure, 0.1% poids/volume de méthyle-4-hydroxybenzoate, 0.1% poids/volume de propyle-4-hydroxybenzoate, 5.0% poids/volume de polyvinylepyrrolidone, 65% poids/volume de N-méthyle-2-pyrrolidone, et de l'eau ad inject.au 100% poids/volume.

3. L'emploi d'après la revendication 1, caractérisé en ce que des comprimés pour l'administration orale sont préparés, contenant 25 mg du 2,6-diamino-3-phényle-azopyridine-hydrochlorure, 150 mg de lactose, 70 mg d'amidon de maîs, 20 mg de polyvinylepyrrolidone,8mg de silice colloidale anhydrique et 8mg de stéarate de magnesium.

4. L'emploi d'après la revendication 1, caractérisé en ce qu'un onguent pour l'administration à la peau est préparé, contenant 2.0% poids/volume du 2,6-diamino-3-phényle-azopyridine-hydrochlorure, 0.1% poids/volume de méthyle-4-hydroxybenzoate, 0.03% poids/volume de propyle-4-hydroxybenzoate, 4.0% poids/volume de polyvinylepyrrolidone, 60% poids/volume de propylèneglycol, 1.4% poids/volume de hydroxyéthylecellulose et de l'eau purifié jusqu'à 100% poids/volume.

Formulae.

Form. I

Form. Il